# FASCICULE DE BREVET EUROPEEN

(11) **EP 1 703 837 B1**
(45) Date de publication et mention de la délivrance du brevet: **18.03.2009**
(21) Numéro de dépôt: 04817613.5
(22) Date de dépôt: 29.12.2004
(51) Int. Cl.: A61B 1/005, A61B 1/04, G02B 6/26, G02B 6/35, A61B 1/00

(54) **TETE OPTIQUE MINIATURE A BALAYAGE INTEGRE POUR LA REALISATION D' UNE IMAGE CONFOCALE HOMOGENE, ET SYSTEME D' IMAGERIE CONFOCALE UTILISANT LADITE TETE**
INTEGRIERTER OPTISCHER MINIATURISIERTER ABTASTKOPF ZUR ERZEUGUNG EINES HOMOGENEN KONFOKALEN BILDS UND DIESEN VERWENDENDES KONFOKALES ABBILDUNGSSYSTEM
INTEGRATED SCANNING MINIATURE OPTICAL HEAD FOR PRODUCING A HOMOGENEOUS CONFOCAL IMAGE, AND CONFOCAL IMAGING SYSTEM USING SAME

(30) Priorité: 31.12.2003 FR 0315627
(43) Date de publication de la demande: 27.09.2006
(73) Titulaire: Mauna Kea Technologies, 75010 Paris (FR)
(72) Inventeur: MATHIEU, Gilles, F-34080 Montpellier (FR); GENET, Magalie, F-78280 Guyancourt (FR); VIELLEROBE, Bertrand, F-94130 Nogent sur Marne (FR); BERIER, Frédéric, F-29210 Plouarzel (FR)
(74) Mandataire: Pontet, Bernard
(86) Numéro de dépôt international: PCT/FR2004/003402
(87) Numéro de publication internationale: WO 2005/072597

(56) Documents cités:
- EP-A- 0 664 101
- US-A- 4 202 619
- US-A1- 2001 055 462
- US-A1- 2002 197 414
- US-A1- 2004 073 120
- US-A1- 2004 135 886
- US-A1- 2004 151 466
- US-A1- 2004 191 398
- US-B1- 6 485 413
- US-B1- 6 564 087

## Description

La présente invention se rapporte à une tête optique miniature à balayage intégré pour la réalisation d'une image confocale homogène, ainsi qu'à un système d'imagerie confocale utilisant cette tête optique.

Elle trouve une application particulièrement intéressante dans le domaine de l'imagerie confocale haute résolution, permettant d'observer et d'analyser un tissu biologique in vivo in situ, notamment via le canal opérateur d'un endoscope (diamètre intérieur compris entre 2 et 3 mm) ou avec une tête optique intégrée à l'endoscope. L'invention peut également s'appliquer aux domaines de la dermatologie ou de la gynécologie nécessitant une miniaturisation moins poussée de la tête optique, ou encore dans le domaine des analyses biologiques in situ, sur l'homme ou sur le petit animal.

Selon un premier type de système, notamment décrit dans la demande de brevet WO OO/16151, on utilise un guide d'image, constitué d'un faisceau de fibres optiques souples, comportant à son extrémité distale une tête optique de focalisation destinée à venir en contact de l'échantillon à analyser. Les moyens de balayage du faisceau d'excitation sont situés à l'extrémité proximale du guide d'image prévus pour balayer tour à tour les fibres. Le caractère confocal réside ici notamment dans le fait que la même fibre optique du guide est utilisée pour véhiculer le signal d'excitation et le signal de retour émis. Ce type de système présente l'avantage d'une tête optique simplifiée du point de vue mécanique et comportant ainsi essentiellement des moyens optiques de focalisation pouvant être miniaturisés. En revanche, il présente certains inconvénients, liés à l'utilisation d'une matrice de fibres optiques, notamment le problème de l'échantillonnage du tissu (continuité entre les points d'excitation correspondant à l'éclairage d'une fibre), le problème de l'injection des fibres une à une et des réflexions parasites à l'entrée et à la sortie du guide d'image en ce qui concerne notamment la rétrodiffusion, le traitement sophistiqué informatique de l'image nécessaire pour corriger ensuite le motif des fibres sur l'image, etc.

Selon un autre type de système connu, les moyens de balayage du faisceau sont situés dans la tête optique à l'extrémité distale d'une unique fibre optique souple. Le caractère confocal est ici obtenu grâce au fait que la fibre optique est utilisée pour véhiculer le signal d'excitation et de retour émis avec un diamètre de coeur de la fibre et une ouverture numérique appropriés.

Les inconvénients de ce type de système sont alors essentiellement liés aux difficultés pour miniaturiser la tête, à la reproductibilité et la fiabilité des moyens mécaniques utilisés pour réaliser le balayage du faisceau émergent de la fibre optique.

Le document US 6,091,067 décrit un système de balayage dans lequel une fibre optique est fixée à deux cales piézoélectriques bimorphes, l'une des cales est placée dans la direction axiale de la fibre et l'autre dans la direction perpendiculaire à l'axe optique. Les cales, pour offrir un débattement approprié par rapport au champ d'observation, doivent présenter une longueur donnée. Perpendiculairement à l'axe de la fibre optique, cette contrainte de longueur conduit en fait à un diamètre de tête optique trop important pour les applications in vivo in situ visées selon la présente invention.

Plusieurs documents décrivent des têtes optiques miniatures confocales mettant en oeuvre des micro miroirs de type micromécaniques (MEMs).

La demande de brevet US 2002/0018276 décrit un système confocal miniature utilisant une fibre optique. La lumière sortant de la fibre est réfléchie sur la partie métallisée d'une lentille. Cette lumière vient ensuite se réfléchir sur un micro miroir MEMs à deux dimensions entourant la fibre. La lumière est alors envoyée vers l'échantillon via un système optique. La lumière revenant de l'échantillon suit le chemin inverse et repasse par la fibre qui sert de filtrage spatial. Le système est miniature, présentant 2 mm de diamètre et 2,5 mm de longueur.

Les brevets US 6,154,305, US 6,088,145, US 6,007,208, US 5,907,425, US 5,742,419, US 6,172,789 et US 6,057,952 décrivent une tête confocale dans laquelle le balayage du champ d'observation est réalisé par deux micro miroirs MEMs pivotés électrostatiquement. La tête proposée est miniaturisable mais en revanche offre un champ d'observation de 60 x 60 µm trop petit par rapport aux applications visées selon l'invention correspondant à un champ au minimum de 100 x 100 µm pour pouvoir observer par exemple plusieurs noyaux cellulaires de 5 µm de diamètre espacés en général de plusieurs dizaines de µm. Le nombre d'image par seconde de 5 à 8 est par ailleurs encore insuffisant pour une imagerie en temps réel (nécessitant un minimum de 10 à 12 images par secondes en mode le plus lent de 640 lignes). Par ailleurs encore, pour certains d'entre eux, le champ d'observation se trouve parallèle à l'axe de la fibre optique, ce qui peut conduire à des difficultés pratiques d'utilisation (positionnement correct de la sonde).

D'une manière générale, le changement de direction du faisceau optique par des réflexions successives sur des micro-miroirs entraîne des aberrations optiques, notamment de la distorsion ou de la courbure de champ, qu'il est nécessaire de corriger.

Le document US 6,294,775 divulgue un système endoscopique miniature utilisant une fibre optique qui est mise en résonance suivant deux axes. Une optique permet de focaliser le faisceau sortant de la fibre dans l'échantillon. Le balayage a la particularité d'être réalisé en spirale. Toutefois, les images obtenues préservent une qualité inhomogène dans tout le champ d'observation.

Les documents US 6,485,413 B1 et US 2001/0055462 A1 décrivent des têtes de balayage.

La présente invention a pour but de remédier aux inconvénients précités en proposant un système permettant d'obtenir des images homogènes dans tout le champ.

Un autre but de l'invention est de proposer une tête suffisamment miniature pour être intégrée dans le canal opérateur d'un endoscope par exemple.

L'invention a encore pour but un système apte à réaliser une image en temps réel (au moins 10 images par seconde) et couvrir un champ à imager de l'ordre au minimum de 100µm x 100µm et de préférence 150µm x 150µm.

On atteint au moins l'un des buts précités avec une tête optique confocale miniature selon la revendication 1 pour un système d'imagerie confocale, notamment endoscopique, ladite tête comprenant une source ponctuelle pour produire un faisceau lumineux. Selon l'invention, ladite tête optique comprend en outre :
- une lentille boule disposée à l'extrémité de la tête optique, de préférence partiellement à l'extérieur, pour faire converger le faisceau lumineux en un point d'excitation situé dans un champ observé subsurfacique d'un échantillon, l'ouverture numérique de cette lentille et les spécifications (diamètre et ouverture numérique) de la source ponctuelle étant adaptées pour assurer la confocalité de l'ensemble, et
- des moyens de balayage pour déplacer la source ponctuelle en rotation de façon à ce que le point d'excitation balaye le champ observé.

Le balayage consiste en des mouvements de rotation suivant deux axes passant par le centre de la lentille boule.

La lentille boule permet de focaliser le faisceau laser à l'intérieur de l'échantillon. Elle présente de nombreux avantages :
- une symétrie sphérique : cette symétrie associée à un balayage en rotation permet d'obtenir une image homogène puisque les aberrations restent constantes dans tout le champ, contrairement à la plupart des dispositifs de l'art antérieur,
- ouverture numérique importante (ON=1 dans l'air) : cette forte ouverture numérique permet de collecter un maximum de photons provenant du plan de focalisation, et associée au faible diamètre de la source ponctuelle, elle permet d'assurer une bonne confocalité à l'ensemble du système,
- faible diamètre : le diamètre des lentilles boules peut varier de quelques dizaines de micromètres à quelques dizaines de millimètres, ce diamètre qui va dimensionner le système devra être choisi en fonction de la taille du champ à observer et du site que l'on souhaite étudier pour lui conférer un caractère non invasif, et
- facilité de montage : pas de problème de tilt avec une lentille boule que l'on doit placer dans une tête cylindrique.

Avec la tête optique selon l'invention, on atteint une miniaturisation suffisante. En effet, l'utilisation d'une lentille boule et d'une source ponctuelle, à partir d'une seule fibre optique par exemple, permet de réduire l'encombrement du système, et donc le diamètre total tout en conservant une grande ouverture numérique sur l'échantillon et un très bon critère de confocalité.

Le caractère confocal et l'homogénéisation des aberrations dans le champ sont nécessaires pour obtenir une image de bonne qualité et qui ne présente pas de différences entre centre et bord du champ liées au dispositif optique.

Selon une première variante de l'invention, lors du balayage, la source ponctuelle pivote indépendamment de la lentille boule. Dans ce cas, la distance entre la source ponctuelle et le centre de la lentille boule est maintenue constante de sorte que le champ observé est courbe. Les moyens de balayage agissent donc directement sur la source ponctuelle en la déplaçant selon des mouvements de rotation autour d'un hémisphère de la lentille boule. Cette dernière peut avantageusement rester fixe.

Selon une seconde variante de l'invention, lors du balayage, la source ponctuelle est solidaire de la lentille boule. Cette dernière peut donc pivoter suivant deux axes passant par son centre. Avantageusement, les moyens de balayage peuvent directement agir sur la lentille boule. On peut aussi prévoir une action sur la lentille boule et sur la source ponctuelle. Dans ce cas particulier de mode de balayage où la lentille boule pivote, cette lentille boule doit glisser sur l'échantillon car elle bouge par rapport à celui-ci pour réaliser l'image. Cela suppose donc que l'échantillon reste fixe par rapport à la tête et plus particulièrement par rapport à la lentille boule. Pour ce faire, la tête optique selon l'invention peut avantageusement comprendre des moyens pour amener un liquide entre la surface extérieure de la lentille boule et l'échantillon de façon à faciliter le glissement de la lentille boule sur l'échantillon. Ce liquide peut par exemple consister en un film d'eau amené via la tête optique ou formé de manière naturelle. Autrement, on peut prévoir une fine lame courbe rigide, en guise de hublot conçu pour permettre à la lentille boule de glisser sur l'échantillon.

De préférence, la tête optique peut comprendre en outre des moyens optiques correcteurs solidaires de la source ponctuelle et disposés entre cette source ponctuelle et la lentille boule pour corriger des aberrations résiduelles de la lentille boule.

Ainsi, selon le premier mode de balayage où la lentille boule peut rester fixe, la source ponctuelle est solidaire de l'optique correctrice et toutes deux pivotent suivant deux axes de rotation θ et φ par rapport à la lentille boule. La distance entre l'optique correctrice et la lentille boule est maintenue constante au cours du temps. Selon le second mode balayage, l'ensemble source ponctuelle+optique correctrice+lentille boule pivote suivant deux axes de rotation θ et φ avec pour centre de rotation le centre de la lentille boule. Dans ce cas, l'optique correctrice et la lentille boule peuvent être collées.

Dans ces deux cas de figures, les moyens de balayage comprennent avantageusement des moyens pour réaliser des balayages suivant les deux axes de rotation de la lentille boule θ et φ de façon à obtenir une image bidimensionnelle en temps réel. On prévoit alors un balayage à une fréquence sensiblement de 4kHz suivant une direction afin d'assurer une cadence de 10 à 12 images/s.

Par ailleurs, les moyens de balayage sont adaptés pour supporter le déplacement de la source ponctuelle et d'un ou plusieurs systèmes optiques (optique correctrice seule ou optique correctrice + lentille boule). Le balayage micro-mécanique peut être effectué aux moyens de micro-moteurs, de systèmes piézoélectriques, ou de MEM's avec tout type d'actionnement envisageable : électrostatique, magnétique, thermique... Ces moyens de balayage sont réglés avec précision afin d'effectuer un balayage dans un plan hémisphérique qui de préférence suit parfaitement la surface de la lentille boule. Le champ imagé est ainsi courbe.

Selon un mode de réalisation préféré de l'invention, la tête optique comprend la partie terminale d'une fibre optique adaptée à conduire le faisceau lumineux depuis une source extérieure, le faisceau lumineux émergeant de la fibre constituant la source ponctuelle. La fibre optique est de préférence monomode avec un diamètre de coeur et une ouverture numérique permettant un filtrage spatial du signal de retour et donc assurant la confocalité de la tête.

En d'autres termes, la fibre optique est monomode longitudinale pour permettre à la fois que l'illumination de l'échantillon soit la plus homogène possible et que le filtrage spatial au retour soit le meilleur possible. L'ouverture numérique de la fibre optique peut être variable et choisie en fonction du grandissement que l'on souhaite donner au dispositif afin d'optimiser le flux d'excitation.

Suivant une variante, la source ponctuelle est constituée d'une source laser de type VCSEL, présentant une ouverture numérique et un diamètre de sortie de cavité compatible avec un système confocal, et associée à un détecteur placé derrière la cavité du VCSEL.

Suivant un autre aspect de l'invention, il est proposé, un système d'imagerie confocale comportant :
- une tête optique confocale à balayage intégré telle que définie précédemment ;
- une source adaptée à émettre un faisceau lumineux ;
- des moyens de détection d'un signal émis ;
- des moyens électroniques et informatiques de commande et de traitement du signal émis adaptés à reconstruire une image confocale d'un champ imagé.

Le système peut comprendre en outre une fibre optique reliée à une source laser et des moyens de couplage pour coupler ladite fibre avec la fibre optique de transport jusqu'à et depuis la tête optique et une fibre de transport du signal émis jusqu'aux moyens de détection.

Selon l'invention, lorsque la tête optique comprend une source laser VCSEL et un détecteur intégré, le système comprend des moyens de liaison souple entre la tête optique et les moyens de traitement de signal.

D'autres avantages et caractéristiques de l'invention apparaîtront à l'examen de la description détaillée d'un mode de mise en oeuvre nullement limitatif, et des dessins annexés, sur lesquels :
- la figure 1 est un schéma général d'un exemple de système d'imagerie confocale fibrée mettant en oeuvre la tête miniature selon l'invention ;
- la figure 2 est une vue en coupe de côté d'une tête miniature selon un premier exemple de réalisation ;
- la figure 3 est une vue en coupe de la tête miniature de la figure 2 pour deux positions distinctes de balayage ;
- la figure 4 est une vue en coupe de côté d'une tête miniature selon un deuxième exemple de réalisation ; et
- la figure 5 est une vue en coupe de la tête miniature de la figure 4 pour deux positions distinctes de balayage.

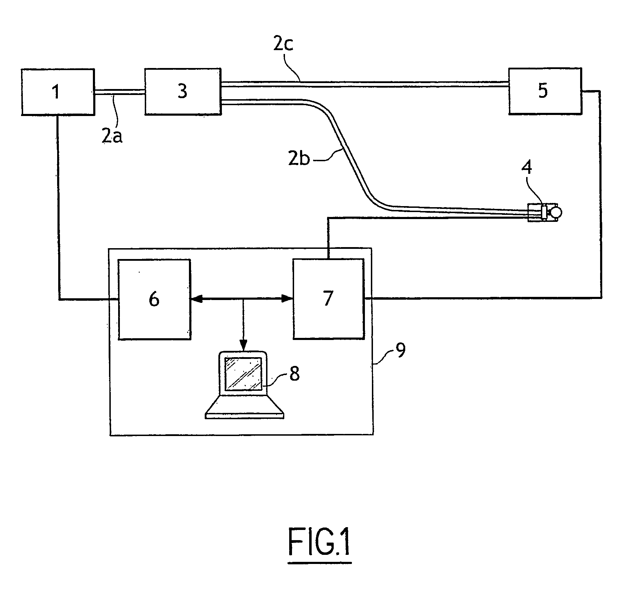

La figure 1 représente schématiquement un système d'imagerie confocale fibrée pouvant inclure une tête miniature selon l'invention.

Le système comprend une source 1, par exemple une source laser, adaptée à émettre un signal d'excitation à une longueur d'onde capable de générer dans un échantillon un signal de retour de fluorescence ou de rétrodiffusion, ledit signal étant transporté par une première fibre optique monomode 2a jusqu'à des moyens de couplage 3, par exemple un coupleur 50/50 de fibre, prévus pour diriger le signal d'excitation provenant de la source 1 dans une fibre optique monomode 2b au bout de laquelle se trouve la tête optique miniature 4 selon l'invention et pour diriger le signal de retour provenant du site excité vers des moyens de détection 5, par exemple un photodétecteur, à l'aide d'une troisième fibre optique monomode 2c. Le système comprend une unité complète 9 de pilotage électronique et informatique dotée des moyens électroniques 6 de contrôle, de commande et de synchronisation, permettant de piloter la source 1, les moyens de balayage de la tête optique 4 et les moyens de détection 5, de manière synchronisée, afin notamment de connaître l'emplacement du signal dans l'échantillon pour permettre la construction d'une image en temps réel. L'unité 9 comprend en outre des moyens électroniques 7 d'amplification, de mise en forme et de conversion A/N du signal détecté par les moyens de détection 5, des moyens informatiques 8 comprenant une carte d'acquisition, une carte graphique et des moyens d'affichage des images obtenues.

Ce système fonctionne globalement de la manière suivante : la tête optique miniature 4 est mise au contact d'un échantillon à analyser, par exemple via le canal opérateur d'un endoscope. La source 1 envoie un signal d'excitation ou faisceau laser à une longueur d'onde choisie dans la portion de fibre 2a. Le coupleur 3 dirige le signal d'excitation dans la portion de fibre 2b conduisant le signal dans la tête optique 4 où il est balayé et focalisé sur une surface d'analyse (ou champ d'analyse) à une profondeur donnée dans l'échantillon. Un signal de retour issu de la surface balayée dans l'échantillon suit le chemin inverse du signal d'excitation jusqu'au coupleur 3 : il est capté par les moyens optiques de la tête optique 4, recouplé dans la portion de fibre 2b, puis dirigé par le coupleur 3 dans la portion de fibre 2c vers le détecteur 5. Le signal détecté est amplifié et converti en un signal numérique, puis traité de manière informatique pour constituer un élément d'image affichée en temps réel.

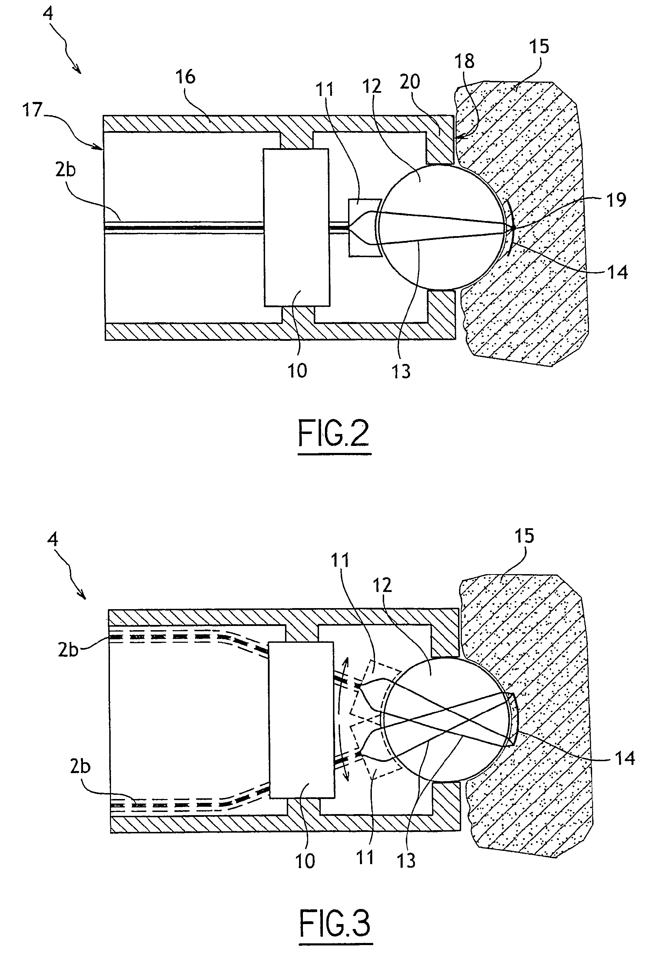

La tête miniature selon l'invention est maintenant décrite en détails en référence aux exemples de réalisation choisis et représentés aux figures 2 à 5.

Sur les figures 2 et 3, on distingue un premier mode de réalisation de la tête optique 4 selon l'invention. Cette tête 4 est une structure de support mécanique constituée d'un corps creux 16, par exemple un porte-optiques tubulaire ouvert à une première extrémité 17 et fermé de façon étanche à une seconde extrémité 18. La fibre optique 2b pénètre jusque dans la tête 4 via l'ouverture 17. L'extrémité de la fibre optique 2b est solidaire d'une optique correctrice 11.

Dans l'axe du faisceau laser 13 sortant de la fibre optique 2b, après l'optique correctrice 11, se trouve une lentille boule 12 permettant de focaliser ce faisceau laser en un point d'excitation, le spot 19, situé dans l'échantillon 15, qui est par exemple le tissu d'un organisme vivant. L'optique correctrice et la lentille boule permettent de focaliser la lumière à une profondeur de quelques dizaines de microns dans l'échantillon.

La lumière issue du tissu 15 repasse dans l'ensemble de la tête optique avant d'être re-couplée dans la fibre optique 2b qui sert de filtrage spatial. Le caractère confocal du dispositif qui consiste à ne détecter que les photons provenant de cette profondeur est assuré par les caractéristiques de la fibre optique 2b et de l'ensemble optique correctrice 11 et lentille boule 12.

La fibre optique est monomode longitudinale pour permettre à la fois que l'illumination du tissu 15 soit la plus homogène possible et que le filtrage spatial au retour soit le meilleur possible. L'ouverture numérique de la fibre est choisie pour permettre une collecte optimisée de photons, et pour permettre, conjointement avec un diamètre de coeur approprié, un couplage du signal de retour dans la fibre optique 2b, et donc un filtrage spatial, qui soit le meilleur possible. Typiquement, l'ouverture numérique varie entre 0,2 et 0,4 en fonction du grandissement que l'on souhaite donner au système, et le diamètre de coeur est compris entre 1 et 2 µm.

L'optique correctrice 11, placée entre la fibre optique 2b et la lentille boule 12, a notamment pour fonction de corriger des aberrations résiduelles de la lentille boule 12 et éventuellement de minimiser les aberrations liées au balayage. Elle peut être constituée d'une ou plusieurs lentilles réfractives (doublets, triplets, lentilles à gradient d'indice... ) ou diffractives. Le nombre de lentilles est relativement limité de sorte que le poids déplacé lors du balayage est faible. Selon le mode de réalisation des figures 2 et 3, l'optique correctrice 11 est solidaire de la fibre optique 2b mais pas de la lentille boule 12.

La lentille boule est calée de façon étanche dans un orifice circulaire réalisé dans le hublot de sortie 20 de la tête optique. Cette lentille boule 12 est partiellement disposée à l'extérieur du corps 16 de sorte que lorsque la tête 4 est posée sur le tissu 15, la partie extérieure de la lentille boule constitue une protubérance s'enfonçant dans le tissu 15.

Pour réaliser une image bidimensionnelle du champ observé 14, on balaye le faisceau laser 13 de façon à ce que le spot 19 décrive ce champ observé 14. Pour ce faire, la tête optique 4 comporte des moyens de balayage 10 supportés par le corps 16 et agencés de façon à déplacer l'ensemble fibre optique 2b-optique correctrice 11 dans un plan hémisphérique. Le champ observé 14 est alors un champ hémisphérique ou plus généralement courbe. L'optique correctrice 12 présente une face qui épouse la forme de la lentille boule 12 sans jamais entrer en contact avec elle. L'inter-espace entre l'optique correctrice 11 et la lentille boule 12 reste constant pendant le balayage. Ce balayage est réalisé suivant deux axes passant par le centre de la lentille boule. Sur la figure 3 on voit deux positions extrêmes de balayage suivant un axe. L'angle de pivotement est choisi de façon à permettre un champ observé 14 de grande dimension et les vitesses de balayage sont telles que les images sont obtenues en temps réel (au moins 10 images par seconde). Le système de balayage peut comporter des moyens piézoélectriques (non représentés) et des moyens MEM's (non représentés) pour réaliser respectivement un balayage rapide suivant un premier axe à une fréquence de sensiblement 4kHz et un balayage lent suivant un second axe perpendiculaire au premier à une fréquence entre 10 et 15 Hz.

L'ensemble des éléments inclus dans la tête optique 4 présentent des dimensions compatibles avec une miniaturisation de la tête qui doit présenter un diamètre total extérieur de 2 à 3 mm maximum. Les éléments actionnés par les moyens de balayage doivent être résistants et capables de répondre à des contraintes mécaniques.

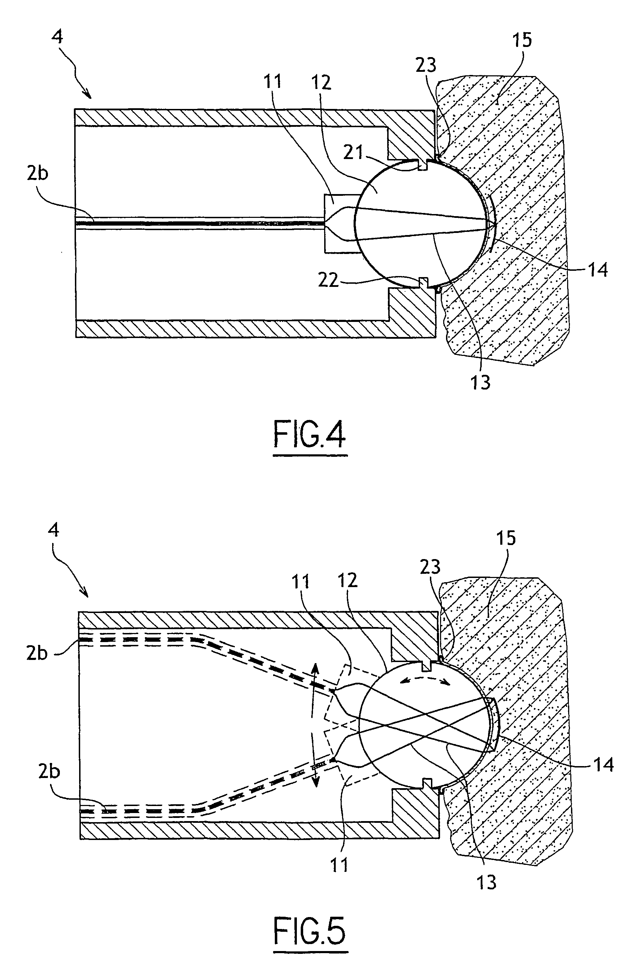

Dans le mode de réalisation des figures 2 et 3, la lentille boule 12 est désolidarisée de la fibre optique 2b et peut ainsi rester fixe. Le second mode de réalisation, représenté sur les figures 4 et 5, consiste au contraire en un ensemble solidaire composé de la fibre optique 2b, de l'optique correctrice 11 et de la lentille boule 12. L'inter-espace entre l'optique correctrice 11 et la lentille boulle 12 est supprimé. Le second mode diffère également du premier mode des figures 2 et 3 par le fait que les moyens de balayage 21 et 22 sont associés au hublot de sortie 20 et font pivoter la lentille boule 12 suivant deux axes de rotation passant par le centre de la lentille boule 12.

Dans ce cas de balayage direct sur la lentille boule 12, on forme un film d'eau 23 sur la face extérieure de la lentille boule de façon à faciliter le glissement sur la surface extérieure du tissu 15. L'eau peut être amenée par un conduit non représenté via la tête optique 4, mais elle peut aussi être formée par d'autres moyens. On peut aussi utiliser à la place ou en combinaison un hublot tel que défini précédemment.

On va maintenant citer ci-après, trois exemples possibles de dimensionnement de la tête miniature à balayage laser selon l'invention :
Exemple 1 :
   - Champ source : 500µm x 500µm
   - Angle de balayage (θ, φ) : +/- 3,7°
   - Ouverture numérique de la fibre optique 2b: ON= 0,25
   - Diamètre de coeur de la fibre optique 2b : ∅_{coeur} = 2,1 µm
   - Diamètre de la lentille boule 12 : ∅_{L} = 2 mm
   - Distance extrémité fibre-centre lentille boule ≤ 3,8 mm
   - Ouverture numérique de la lentille boule : ON_{L} = 1,25 dans l'eau
   - Grandissement du système optique : G = 5
   - Champ imagé 14 dans le tissu 15 : 100µm x 100µm
   - Diamètre du spot 19 focalisé dans le tissu : limité par la diffraction dans tout le champ
Exemple 2 :
   - Champ source : 500µm x 500µm
   - Angle de balayage (θ, φ) : +/- 6,22°
   - Ouverture numérique de la fibre optique 2b : ON= 0,4
   - Diamètre de coeur de la fibre optique 2b : ∅_{coeur} = 1,31µm
   - Diamètre de la lentille boule 12 : ∅_{L} = 2 mm
   - Distance extrémité fibre-centre lentille boule ≤ 2,29 mm
   - Ouverture numérique de la lentille boule : ON_{L} = 1,20 dans l'eau
   - Grandissement du système optique : G = 3
   - Champ imagé 14 dans le tissu 15 : 166µm x 166µm
   - Diamètre du spot 19 focalisé dans le tissu : limité par la diffraction dans tout le champ
Exemple 3 :
   - Champ source : 300µm x 300µm
   - Angle de balayage (θ, φ) : +/- 7,5°
   - Ouverture numérique de la fibre optique 2b : ON= 0,4
   - Diamètre de coeur de la fibre optique 2b : ∅_{coeur} = 1,31µm
   - Diamètre de la lentille boule 12 : ∅_{L} = 1 mm
   - Distance extrémité fibre-centre lentille boule ≤ 1,14 mm
   - Ouverture numérique de la lentille boule : ON_{L} = 1,20 dans l'eau
   - Grandissement du système optique : G = 3
   - Champ imagé 14 dans le tissu 15 : 100µm x 100µm
   - Diamètre du spot 19 focalisé dans le tissu : limité par la diffraction dans tout le champ.

L'exemple 1 par rapport à l'exemple 2 présente un plus fort grandissement et par conséquent de meilleures résolutions latérale et axiale, mais ce au détriment du champ d'observation. L'exemple 3 par rapport à l'exemple 2 possède un champ imagé moins important, mais un encombrement plus faible (∅=1 mm au lieu de 2 mm) et par conséquent une meilleure accessibilité. Un autre avantage de l'invention est que, dans les deux cas de balayage décrits précédemment, l'amplitude de balayage est faible, donc un design plus facile à réaliser. Par conséquent, le dimensionnement du système devra être adapté à l'objet d'étude, au domaine d'application et au mode de fonctionnement qui peut être soit un mode d'imagerie par fluorescence soit un mode d'imagerie en rétrodiffusion.

Le système selon l'invention permet d'obtenir une image confocale en temps réel (environ 10 images/s) de très bonne qualité optique et homogène dans tout le champ; et ce au moyen d'une tête miniature (diamètre de quelques mm) à balayage laser. Une telle configuration doit permettre d'imager des sites difficiles d'accès in vivo sur l'homme ou l'animal sans invasivité (cas endoscopique) ou avec très peu d'invasivité (cas de micro-incisions).

Bien sûr, l'invention n'est pas limitée aux exemples qui viennent d'être décrits et de nombreux aménagements peuvent être apportés à ces exemples sans sortir du cadre de l'invention. En effet, lorsque la source ponctuelle (fibre optique, VCSEL), l'optique correctrice et la lentille boule sont solidaires, on peut envisager des modes de balayage selon lesquels les moyens de balayage sont en prise directe avec la source ponctuelle (premier mode) et/ou avec l'optique correctrice.

## Revendications

1. Tête optique confocale miniature (4) pour un système d'imagerie confocale, notamment endoscopique, ladite tête (4) comprenant :
- une source ponctuelle pour produire un faisceau lumineux (13), et
- une lentille boule (12) disposée à l'extrémité de la tête optique (4), pour faire converger ledit faisceau lumineux (13) en un point d'excitation (19) situé dans un champ observé (14) subsurfacique d'un échantillon (15), l'ouverture numérique de cette lentille et les dimensions de la source ponctuelle étant adaptées pour assurer la confocalité de l'ensemble,
**caractérisée en ce qu'**elle comprend en outre des moyens de balayage (10, 21, 22) pour déplacer la source ponctuelle en rotation suivant deux axes passant par le centre de la lentille boule, de façon à ce que le point d'excitation (19) balaye ledit champ observé, cette lentille boule (12) est partiellement disposée à l'extérieur du corps (16) constituant la tête optique (4) de sorte que lorsque cette tête optique (4) est posée sur l'échantillon (15), la partie extérieure de la lentille boule constitue une protubérance s'enfonçant dans l'échantillon (15).

2. Tête optique selon la revendication 1, **caractérisée en ce que,** lors du balayage, la source ponctuelle pivote indépendamment de la lentille boule..

3. Tête optique selon la revendication 2, **caractérisée en ce que,** lors du balayage, la distance entre la source ponctuelle et le centre de la lentille boule est maintenue constante de sorte que le champ observé (14) est courbe.

4. Tête optique selon la revendication 1, **caractérisée en ce que,** lors du balayage, la source ponctuelle est solidaire de la lentille boule.

5. Tête optique selon la revendication 4, **caractérisée en ce qu'**elle comprend en outre des moyens pour amener un liquide (23) entre la surface extérieure de la lentille boule et l'échantillon de façon à faciliter le glissement de la lentille boule sur l'échantillon.

6. Tête optique selon la revendication 4, **caractérisée en ce qu'**elle comprend en outre une fine lame courbe rigide, en guise de hublot conçu pour permettre à la lentille boule de glisser sur l'échantillon.

7. Tête optique selon l'une quelconque des revendications 4 à 6, **caractérisée en ce que** les moyens de balayage (21, 22) agissent directement sur la lentille boule.

8. Tête optique selon l'une quelconque des revendications précédentes, **caractérisée en ce que** les moyens de balayage (10) agissent directement sur la source ponctuelle.

9. Tête optique selon l'une quelconque des revendications précédentes, **caractérisée en ce qu'**elle comprend en outre des moyens optiques correcteurs (11) solidaires de la source ponctuelle et disposés entre cette source ponctuelle et la lentille boule (12) pour corriger des aberrations résiduelles de la lentille boule.

10. Tête optique selon l'une quelconque des revendications précédentes, **caractérisée en ce que** les moyens de balayage comprennent des moyens pour réaliser des balayages suivant deux axes de rotation de la lentille boule de façon à obtenir une image bidimensionnelle en temps réel.

11. Tête optique selon la revendication 10, **caractérisée en ce que** le balayage suivant un des axes de rotation atteint une fréquence sensiblement de 4 kHz.

12. Tête optique selon l'une quelconque des revendications précédentes, **caractérisée en ce que** les moyens de balayage comprennent des micro-moteurs.

13. Tête optique selon l'une quelconque des revendications précédentes, **caractérisée en ce que** les moyens de balayage comprennent des éléments piézoélectriques.

14. Tête optique selon l'une quelconque des revendications précédentes, **caractérisée en ce que** les moyens de balayage comprennent des moyens micromécaniques de type MEM's.

15. Tête optique selon l'une quelconque des revendications précédentes, **caractérisée en ce qu'**elle comprend la partie terminale d'une fibre optique adaptée à conduire le faisceau lumineux depuis une source extérieure, le faisceau lumineux émergent de la fibre constituant la source ponctuelle.

16. Tête optique selon la revendication 15, **caractérisée en ce que** la fibre optique est monomode avec un diamètre de coeur et une ouverture numérique permettant un filtrage spatial du signal de retour et donc assurant la confocalité de la tête,

17. Tête optique selon l'une quelconque des revendications 1 à 14, **caractérisée en ce que** la source ponctuelle est constituée d'une source laser de type VCSEL, présentant une ouverture numérique et un diamètre de sortie de cavité compatible avec un système confocal, et associée à un détecteur placé derrière la cavité du VCSEL.

18. Système d'imagerie confocale comportant :
- une tête (4) optique confocale à balayage intégré;
- une source (1,2a,2b) adaptée à émettre un faisceau lumineux ;
- des moyens de détection (5) d'un signal émis ;
- des moyens (9) électroniques et informatiques de commande et de traitement du signal émis adaptés à reconstruire une image confocale d'un champ imagé,
**caractérisé en ce que** la tête optique (4) est selon l'une quelconque des revendications précédentes.

19. Système selon la revendication 18, **caractérisé par** une fibre optique (2a) reliée à une source laser (1) et des moyens de couplage (3) pour coupler ladite fibre (2a) avec la fibre optique de transport (2b) jusqu'à et depuis la tête optique (4) et une fibre de transport (2c) du signal émis jusqu'aux moyens de détection.

20. Système selon la revendication 18, **caractérisé en ce que,** la tête optique comprenant une source laser VCSEL et un détecteur intégré, le système comprend des moyens de liaison souple entre la tête optique et les moyens de traitement de signal.

## Claims

1. Miniature confocal optical head (4) for a confocal imaging system, in particular endoscopic, said head (4) comprising:
- a point source for producing a light beam (13), and
- a ball lens (12) arranged at the end of the optical head (4), in order to cause said light beam (13) to converge into an excitation point (19) situated in a subsurface field under observation (14) of a sample (15), the numerical aperture of this lens and the dimensions of the point source being suitable to ensure the confocality of the assembly,
**characterized in that** it also comprises scanning means (10, 21, 22) for displacing the point source in rotation along two axes passing through the centre of the ball lens, so that the excitation point (19) scans said field under observation, said ball lens (12) is partially arranged outside the body (16) constituting the optical head (4) such that when this optical head (4) is positioned on the sample (15), the outer part of the ball lens constitutes a protuberance pushing into the sample (15).

2. Optical head according to claim 1, **characterized in that,** during scanning, the point source pivots independently of the ball lens.

3. Optical head according to claim 2, **characterized in that,** during scanning, the distance between the point source and the centre of the ball lens is kept constant so that the field under observation (14) is curved.

4. Optical head according to claim 1, **characterized in that,** during scanning, the point source is integral with the ball lens.

5. Optical head according to claim 4, **characterized in that** it also comprises means for introducing a liquid (23) between the external surface of the ball lens and the sample so as to ease the sliding of the ball lens over the sample.

6. Optical head according to claim 4, **characterized in that** it also comprises a fine rigid curved plate used as a window designed to allow the ball lens to slide over the sample.

7. Optical head according to any one of claims 4 to 6, **characterized in that** the scanning means (21, 22) act directly on the ball lens.

8. Optical head according to any one of the preceding claims, **characterized in that** the scanning means (10) act directly on the point source.

9. Optical head according to any one of the preceding claims, **characterized in that** it also comprises corrective optical means (11) integral with the point source and arranged between this point source and the ball lens (12) in order to correct residual aberrations of the ball lens.

10. Optical head according to any one of the preceding claims, **characterized in that** the scanning means comprise means for carrying out scanning along two rotational axes of the ball lens so as to obtain a two-dimensional image in real time.

11. Optical head according to claim 10, **characterized in that** the scanning along one of the rotational axes reaches a frequency of approximately 4 kHz.

12. Optical head according to any one of the preceding claims, **characterized in that** the scanning means comprise micro-motors.

13. Optical head according to any one of the preceding claims, **characterized in that** the scanning means comprise piezoelectric elements.

14. Optical head according to any one of the preceding claims, **characterized in that** the scanning means comprise MEMs-type micromechanical means.

15. Optical head according to any one of the preceding claims, **characterized in that** it comprises the terminal part of an optical fibre suitable for conducting the light beam from an external source, the light beam emerging from the fibre constituting the point source.

16. Optical head according to claim 15, **characterized in that** the optical fibre is monomode with a core diameter and a numerical aperture allowing a spatial filtering of the return signal and therefore ensuring the confocality of the head.

17. Optical head according to any one of claims 1 to 14, **characterized in that** the point source is constituted by a VCSEL-type laser source, having a numerical aperture and a cavity outlet diameter compatible with a confocal system, and associated with a detector placed behind the VCSEL cavity.

18. Confocal imaging system comprising:
- a confocal optical head (4) with integrated scanning;
- a source (1, 2a, 2b) suitable for emitting a light beam;
- means of detection (5) of an emitted signal;
- means (9) for electronic and computer control and processing of the signal emitted suitable for reconstructing a confocal image of a field image,
**characterized in that** the optical head (4) is according to any one of the preceding claims.

19. System according to claim 18, **characterized by** an optical fibre (2a) connected to a laser source (1) and coupling means (3) for coupling said fibre (2a) with the optical fibre (2b) for transport to and from the optical head (4) and a fibre (2c) for transporting the emitted signal to the detection means.

20. System according to claim 18, **characterized in that,** the optical head comprising a VCSEL laser source and an integrated detector, the system comprises flexible connection means between the optical head and the signal processing means.

## Patentansprüche

1. Miniaturisierter, konfokaler, optischer Kopf (4) für ein konfokales Abbildungssystem, insbesondere ein endoskopisches, wobei der Kopf (4) Folgendes aufweist:
- eine punktförmige Quelle zum Erzeugen eines Lichtstrahls (13) und
- eine Kugellinse (12), die an dem Ende des optischen Kopfs (4) angeordnet ist, um den Lichtstrahl (13) in einem Erregungspunkt (19), der sich in einem beobachteten Feld (14) unter der Oberfläche einer Probe (15) befindet, konvergieren zu lassen, wobei die numerische Apertur dieser Linse und die Maße der punktförmigen Quelle angepasst sind, um die Konfokalität der Einheit sicherzustellen,
**dadurch gekennzeichnet, dass** er ferner Abtastmittel (10, 21, 22) aufweist, um die punktförmige Quelle in Drehung entlang von zwei Achsen, die durch die Mitte der Kugellinse verlaufen, derart zu bewegen, dass der Erregungspunkt (19) das Feld abtastet, wobei diese Kugellinse (12) teilweise außerhalb des Körpers (16), der den optischen Kopf (4) bildet, derart angeordnet ist, dass der äußere Teil der Kugellinse, wenn dieser optische Kopf (4) auf der Probe (15) aufliegt, einen Vorsprung bildet, der sich in die Probe (15) drückt.

2. Optischer Kopf nach Anspruch 1, **dadurch gekennzeichnet, dass** die punktförmige Quelle beim Abtasten unabhängig von der Kugellinse schwenkt.

3. Optischer Kopf nach Anspruch 2, **dadurch gekennzeichnet, dass** die Entfernung zwischen der punktförmigen Quelle und der Mitte der Kugellinse beim Abtasten derart konstant gehalten wird, dass das beobachtete Feld (14) gewölbt ist.

4. Optischer Kopf nach Anspruch 1, **dadurch gekennzeichnet, dass** die punktförmige Quelle beim Abtasten fest mit der Kugellinse verbunden ist.

5. Optischer Kopf nach Anspruch 4, **dadurch gekennzeichnet, dass** er ferner Mittel aufweist, um eine Flüssigkeit (23) zwischen die Außenfläche der Kugellinse und die Probe derart zu bringen, dass das Gleiten der Kugellinse auf der Probe erleichtert wird.

6. Optischer Kopf nach Anspruch 4, **dadurch gekennzeichnet, dass** er ferner eine feine, gebogene, starre Klinge als Fenster aufweist, das konzipiert ist, um es der Kugellinse zu erlauben, auf der Probe zu gleiten.

7. Optischer Kopf nach einem der Ansprüche 4 bis 6, **dadurch gekennzeichnet, dass** die Abtastmittel (21, 22) direkt auf die Kugellinse wirken.

8. Optischer Kopf nach einem der vorhergehenden Ansprüche, **dadurch gekennzeichnet, dass** die Abtastmittel (10) direkt auf die punktförmigen Quelle wirken.

9. Optischer Kopf nach einem der vorhergehenden Ansprüche, **dadurch gekennzeichnet, dass** er ferner optische Korrekturmittel (11) aufweist, die fest mit der punktförmigen Quelle verbunden und zwischen dieser punktförmigen Quelle und der Kugellinse (12) angeordnet sind, um restlichen Abbildungsfehler der Kugellinse zu korrigieren.

10. Optischer Kopf nach einem der vorhergehenden Ansprüche, **dadurch gekennzeichnet, dass** die Abtastmittel Mittel aufweisen, um Abtastungen entlang von zwei Drehachsen der Kugellinse derart auszuführen, dass man in Echtzeit ein zweidimensionales Bild erzielt.

11. Optischer Kopf nach Anspruch 10, **dadurch gekennzeichnet, dass** das Abtasten entlang einer der Drehachsen eine Frequenz von in etwa 4 kHz erreicht.

12. Optischer Kopf nach einem der vorhergehenden Ansprüche, **dadurch gekennzeichnet, dass** die Abtastmittel Mikromotoren aufweisen.

13. Optischer Kopf nach einem der vorhergehenden Ansprüche, **dadurch gekennzeichnet, dass** die Abtastmittel piezoelektrische Elemente aufweisen.

14. Optischer Kopf nach einem der vorhergehenden Ansprüche, **dadurch gekennzeichnet, dass** die Abtastmittel mikromechanische Mittel des Typs MEM aufweisen.

15. Optischer Kopf nach einem der vorhergehenden Ansprüche, **dadurch gekennzeichnet, dass** er den Endteil einer Lichtleitfaser aufweist, die den Lichtstrahl von einer externen Quelle leiten kann, wobei der Lichtstrahl, der aus der Lichtleitfaser austritt, die punktförmige Quelle bildet.

16. Optischer Kopf nach Anspruch 15, **dadurch gekennzeichnet, dass** die Lichtleitfaser eine Monomodefaser mit einem Kerndurchmesser und einer numerischen Apertur ist, die ein räumliches Filtern des Rückmeldesignals erlaubt und daher die Konfokalität des Kopfes sicherstellt.

17. Optischer Kopf nach einem der Ansprüche 1 bis 14, **dadurch gekennzeichnet, dass** die punktförmige Quelle aus einer Laserquelle des Typs VCSEL besteht, die eine numerische Apertur und einen Hohlraumausgangsdurchmesser aufweist, der mit einem konfokalen System kompatibel ist, und die mit einem Detektor verbunden ist, der hinter dem VCSEL-Hohlraum platziert ist.

18. Konfokales Abbildungssystem, das Folgendes aufweist:
- einen konfokalen optischen Kopf (4) mit integrierter Abtastung;
- eine Quelle (1, 2a, 2b), die einen Lichtstrahl abgeben kann;
- Mittel (5) zum Erfassen eines emittierten Signals;
- Elektronik- und EDV-Mittel (9) zum Steuern und Verarbeiten des emittierten Signals, die ein konfokales Bild eines abgebildeten Felds rekonstruieren können,
**dadurch gekennzeichnet, dass** der optische Kopf (4) einem der vorhergehenden Ansprüche entspricht.

19. System nach Anspruch 18, **gekennzeichnet durch** eine Lichtleitfaser (2a), die mit einer Laserquelle (1) verbunden ist, und **durch** Koppelmittel (3) zum Koppeln der Faser (2a) mit der Transportlichtleitfaser (2b) bis zum optischen Kopf (4) und von ihm ausgehend, und eine Faser (2c) zum Transportieren des emittierten Signals bis zu den Detektionsmitteln.

20. System nach Anspruch 18, **dadurch gekennzeichnet, dass** das System, weil der optische Kopf eine VCSEL-Laserquelle und einen integrierten Detektor aufweist, Mittel zum biegsamen Verbinden zwischen dem optischen Kopf und den Signalverarbeitungsmitteln aufweist.
